# EUROPEAN PATENT APPLICATION

(11) **EP 4 353 737 A1**
(43) Date of publication of application: **17.04.2024**
(21) Application number: 22200692.6
(22) Date of filing: 10.10.2022
(51) Int. Cl.: C07K 14/005, C07K 14/47, C07K 16/00, C12N 15/86

(54) **FUSION PROTEINS COMPRISING A CELL SURFACE MARKER SPECIFIC VHH**

(71) Applicant: Universitätsklinikum Hamburg-Eppendorf, 20246 Hamburg (DE)
(72) Inventor: NOLTE, Friedrich, 20246 Hamburg (DE); MANN, Anna Marei, 20246 Hamburg (DE); SCHÄFER, Waldemar, 20246 Hamburg (DE)
(74) Representative: Uexküll & Stolberg

(57) **Abstract**

The invention relates to an adeno-associated virus (AAV) capsid fusion protein comprising comprises an insert comprising a binding domain for a cell surface molecule, in particular, a variable immunoglobulin domain of a heavy chain antibody (VHH), which is inserted in the GH12-GH13 loop of the AAV capsid protein. Further, the invention relates to an AAV capsid fusion protein comprising at least one, preferably at least two VHHs, which are inserted in the AAV capsid protein. Also encompassed is a recombinant adeno-associated virus (AAV) which comprises said AAV capsid fusion protein. Also nucleotide sequences encoding such fusion proteins and vectors including such nucleotide sequences are contemplated. Moreover the invention refers to uses of the fusion proteins, nucleic sequences and corresponding AAVs for use as a medicament.

## Description

### FIELD OF THE INVENTION

The invention relates to an adeno-associated virus (AAV) capsid fusion protein comprising a surface marker binding domain (SMBD), in particular, a variable immunoglobulin domain of a heavy chain antibody (VHH), which is inserted in the GH12-GH13 loop of the AAV capsid protein. Further, the invention relates to an AAV capsid fusion protein comprising at least one, preferably at least two SMBDs, such as two VHHs, which are inserted in the AAV capsid protein. Also encompassed is a recombinant adeno-associated virus (AAV) which comprises said AAV capsid fusion protein. Also nucleotide sequences encoding such fusion proteins and vectors including such nucleotide sequences are contemplated. Moreover the invention refers to uses of the fusion proteins, nucleic sequences and corresponding AAVs for use as a medicament.

### BACKGROUND OF THE INVENTION

Adeno-associated viruses (AAVs) are of increasing importance for gene and tumor therapy. AAVs are used as vectors, since they are non-pathogenic, show low immunogenicity, and confer long-term expression of the transferred gene.

A challenge in the use of AAVs as vectors in gene therapy is their broad tropism, i.e. the parallel infection of several tissues and cell types. Moreover, neutralizing antibodies that occur in about 80 % of the population impede the efficient binding and transduction of target cells.

Current techniques of improving target specificity of AAVs include the chemical conjugation of two Fab fragments (Bartlett et al. 1999), the insertion of peptides into the capsid proteins, the fusion of DARPins to the N-terminus of the VP2 capsid protein (Münch et al. 2013), the use of AAV serotypes that are not recognized by neutralizing antibodies.

AAV2 including VHH in the GH2-GH3 loop have been described in WO2019/211401A1.

However, there is a still a need to increase the AAV portfolio for efficient cell targeting and to improve AAVs for therapy, in particular to improve tissue specificity of AAVs.

### OBJECTIVES AND SUMMARY OF THE INVENTION

Thus, a first aspect of the invention refers to adeno-associated virus (AAV) capsid fusion protein comprising a surface marker binding domain (SMBD), in particular a variable immunoglobulin domain of a heavy chain antibody (VHH) which is inserted in the GH12-GH13 loop of the AAV capsid protein.

Providing a new site for target-specific inserts, such as target specific SMBD (for example VHH), allows the combination with other capsid modifications, including but not limited to point mutations, peptide insertions, ligand insertions, and/or capsid shuffling. Thereby, simultaneous targeting of two different molecules, in particular cell surface molecules, such as two different receptors that are expressed by the target tissue or target cell can be achieved.

Surprisingly, insertion of the SMBD (e.g. VHH) sequence into the GH12-GH13 loop did not impair the structure and function of the AAV capsid fusion protein, such that AAV containing said fusion protein comprising the SMBD (e.g. VHH) in the GH12-GH13 loop can be successfully assembled. This is surprising, since the VHH sequence has a considerable length of over 100 aa, thus it was not clear whether an insert of such length would not disturb the structural and functional integrity of the VP1 and the correspondingly assembled AAV.

Another aspect of the invention refers to an AAV capsid fusion protein comprising at least two, such as two SMBDs, for example at least two VHHs, such as two VHHs.

The incorporation of two VHHs into the AAV allows simultaneous targeting of two receptors that are co-expressed by the target cell.

Surprisingly, the AAV capsid fusion protein can incorporate two SMDCs (for example two VHHs) and still is able to assemble functional AAV particles. Thereby targeting of two different cell surface molecules via VHHs becomes feasible.

Further aspects relate to a recombinant adeno-associated virus (AAV) which comprises said AAV capsid fusion protein. Also nucleotide sequences encoding such fusion proteins and vectors including such nucleotide sequences are contemplated. Moreover, the invention refers to uses of the fusion proteins, nucleic sequences and corresponding AAVs for use as a medicament.

### FIGURE LEGENDS

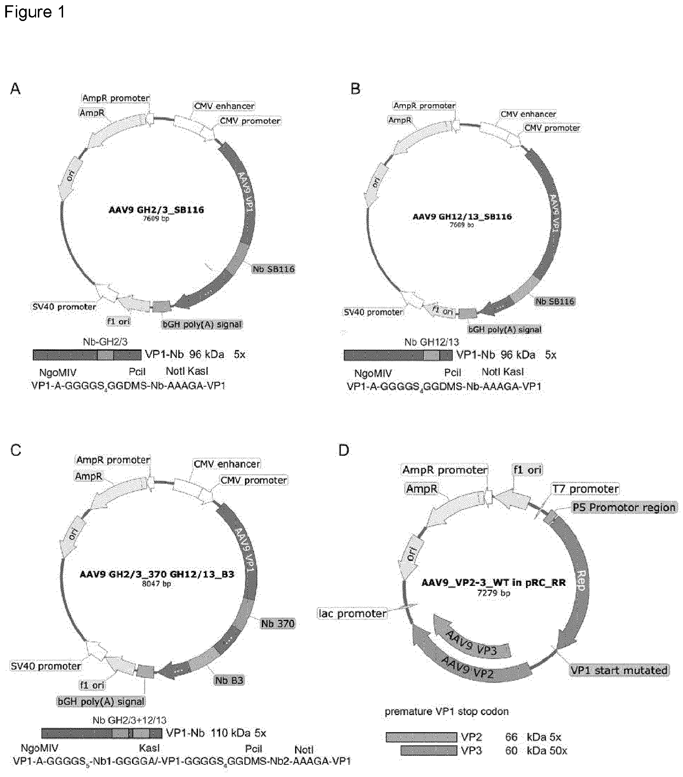

**Figure 1** illustrates the DNA plasmids used for the production of nanobody-carrying AAV capsids, where a membrane-protein-specific nanobody is inserted into prominent surface loops of AAV9. Schematics of the plasmids for the generation of AAV9 that contain a membrane protein-specific VHH in the GH2/3 loop (between amino acids 454 and 455), the GH12/13 loop (between amino acids 587 and 588) or both loops of the VP1 capsid protein of AAV9. AAV capsids consist of 60 capsid proteins (~50 VP3, ~5 VP2, ~5 VP1). **A-C)** pcDNA3.1 plasmids encoding codon-optimized sequences of VP1 of AAV9 with a VHH (CD73-specific SB116) inserted in the GH2/3-loop **(A),** the GH12/13-loop **(B)** or two VHHs (CD38-specific 370 and PD-L1-specific B3) in both loops, respectively **(C).** The Nb insertion site is flanked on the 5` (N-terminal) side by a NgoMIV restriction site coding for two amino acids (AG), a 22 amino acid linker peptide (GGGGS₄ GG) and a Pcil site encoding three amino acid residues (DMS), and on the 3' (C-terminal) side by a short AAAGA linker (encoded by Notl and Kasl restriction sites).
   **D)** pRepCap plasmid encoding VP2 and VP3 and containing a premature stop codon at the sixth codon of the VP1 open reading frame to prevent the expression of unmodified VP1.

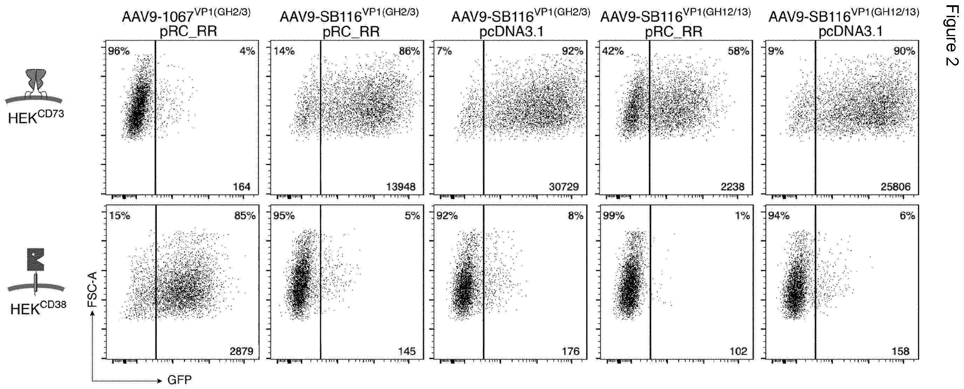

**Figure 2** shows flow cytometry analyses of GFP-expression (numbers indicate the mean fluorescence intensity of all single cells) as a parameter of transduction efficiency two days after treatment of HEK cells stably transfected with human CD38 or mouse CD73 with GFP-encoding AAV. AAV that carry nanobodies in the GH2/3 or GH12/13 loop enhance the transduction of HEK cells expressing the cognate membrane protein 20-100fold compared to the respective unrelated HEK cells. Use of the pcDNA3.1 plasmid during AAV production, that encodes a codon-optimized VP1-Nb fusion protein under the CMV promotor, leads to an even stronger increase in transduction of the target cells (>100fold increase in mean fluorescence intensity).
   HEK cells stably transfected with mouse CD73 (HEK^{CD73}) or human CD38 (HEK^{CD38}) were seeded in 96-well plates (2×10⁴ cells/well) and incubated with 10 µl cell supernatant containing GFP-encoding AAV. 48 h later the transduction efficiency was analyzed via flow cytometric measurement of GFP expression.

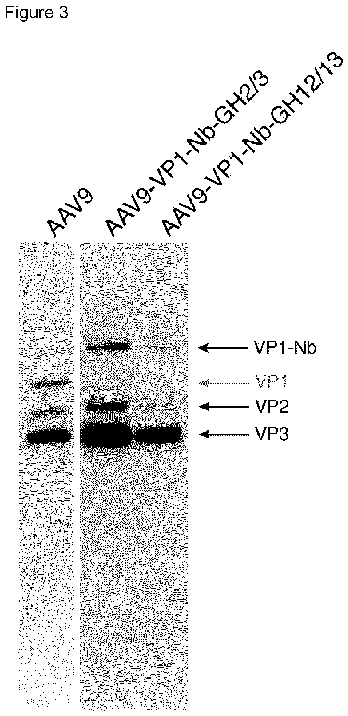

**Figure 3** shows a Western blot analysis of the capsid protein composition of an unmodified AAV9, and of AAV9 that carry a nanobody in the GH2/3 or in the GH12/13 loop. The higher apparent molecular weight of the VP1-Nb fusion proteins reflect the successful insertion of the nanobody (~15 kDa) and demonstrate that the Nanobody-VP1 fusion proteins are incorparated into the virions. Capsid proteins in Western blots of AAV-containing cell supernatants were detected with mAb B1 that recognizes a common epitope near the C-terminus of all capsid proteins. The three capsid proteins are assembled at a ratio of ~ 50 VP3 (60 kDa) : 5 VP2 (70 kDa) : 5 VP1 (85 kDa). As expected, the VP1-Nb-GH2/3 or VP1-Nb-GH12/13 fusion migrates at a higher Mr (100 kDa) than VP1. Nb = CD73-specific nanobody SB116.

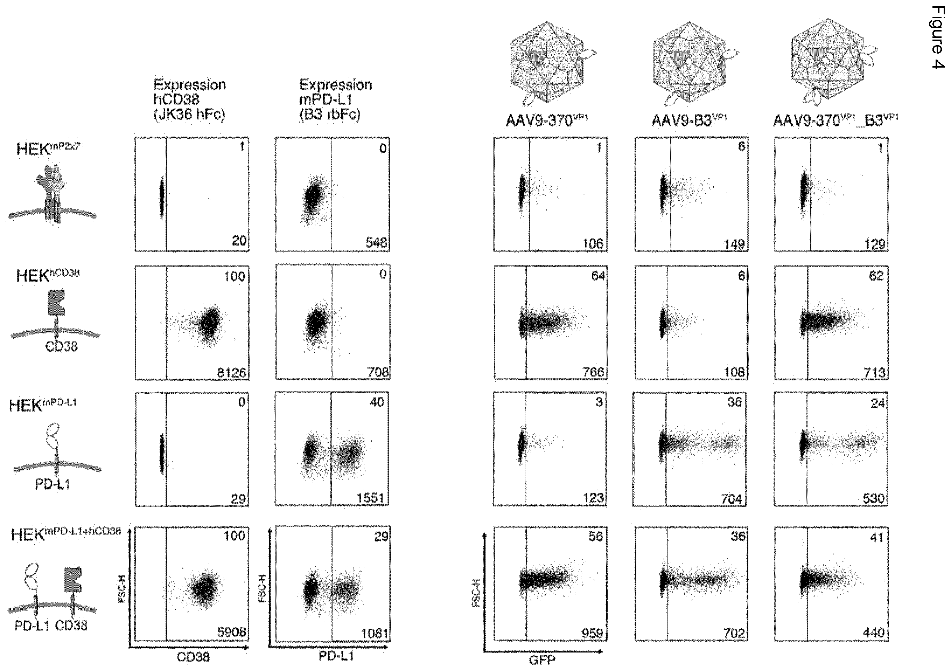

**Figure 4** shows the transduction of HEK cells expressing a single membrane protein (P2X7, CD38 or PD-L1) or expressing two targets (CD38 and PD-L1) with monospecific AAV (AAV9-370^{VP1}, and AAV9-B3 ^{VP1}) and a bispecific AAV (AAV9-370 ^{VP1}_B3 ^{VP1}). Both monospecific AAV enhance the transduction of HEK cells expressing either the cognate membrane protein alone or both targets compared to unrelated control cells (HEK^{P2X7}). The bispecific AAV (AAV9-370 ^{VP1}_B3 ^{VP1}) transduces cells that express only one of the two targets as well as cells that express both targets, demonstrating that both nanobodies - inserted in the same VP1 protein - bind their respective target antigens. HEK cells stably transfected with human CD38 (HEK^{hCD38}) and untransfected HEK cells were transiently transfected with murine PD-L1 (HEK^{mPD-L1}, HEK^{hCD38+mPD-L1}) and seeded onto a 96-well plate, in parallel with HEK cells stably transfected with human CD38 alone (HEK^{CD38}) or mouse P2X7 (HEK^{mP2X7}) (2×10^4 cells/well). The cells were incubated with GFP-encoding AAV that carry a mPD-L1 (B3) or hCD38-specific nanobody (370) inserted into the GH2/3 loop, or both nanobodies inserted into GH2/3-loop (370) and GH12/13-loop (B3). 48h later the transduction efficiency was analyzed via flow cytometric measurement of GFP expression.

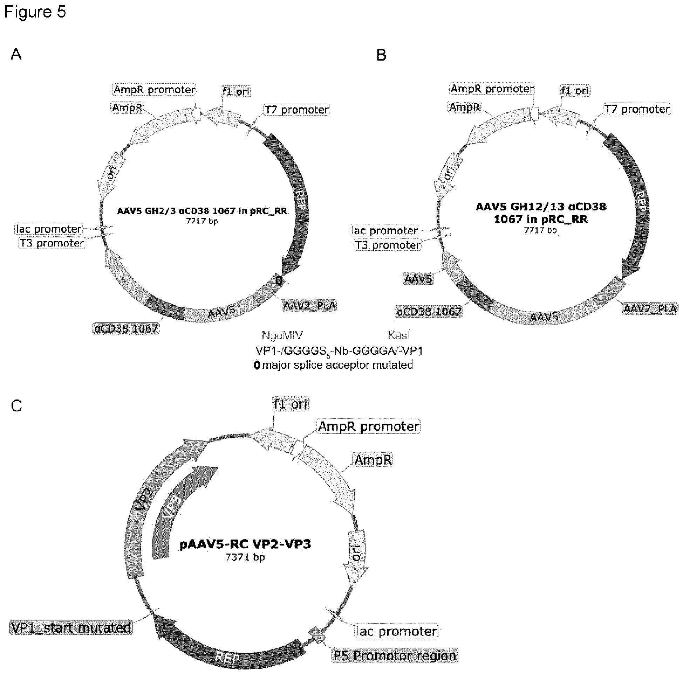

**Figure 5** illustrates the DNA plasmids used for the production of AAV5 capsids, where a membrane protein-specific nanobody is genetically inserted into an exposed surface loop of AAV5 at position 454/455 (GH2/3 loop) or 587/588 (GH12/13 loop) of the viral protein 1 (VP1).
   Schematics of the plasmids for the generation of AAV5 that contain a membrane protein-specific nanobody in the GH2/3 loop (replacing amino acids N443 and T444) or the GH12/13 loop (replacing amino acids S576 and T577) of the VP1 capsid protein of AAV5. **A)** pRC_RR plasmids encoding viral replication proteins (REP) and VP1 of AAV5 with the Nb (CD38-specific 1067) inserted in the GH2/3-loop (left) or the Nb inserted in the GH12/13-loop (right). The nanobody insertion site is flanked on the 5' (N-terminal) side by a NgoMIV restriction site (encoding AG) and a 25 amino acid GGGGS₅ linker peptide, and on the 3' (C-terminal) side by a GGGGA linker, encompassing a Kasl restriction site. The first 137 amino acids of VP1 originate from AAV2 for improved compatibility with the AAV2-derived REP proteins and inverted terminal repeats (ITRs) on the transgene. **B)** pAAV5-RC_VP2-3 plasmid encoding VP2 and VP3 of AAV5 with mutated VP1 start codon to inhibit the expression of unmodified VP1.

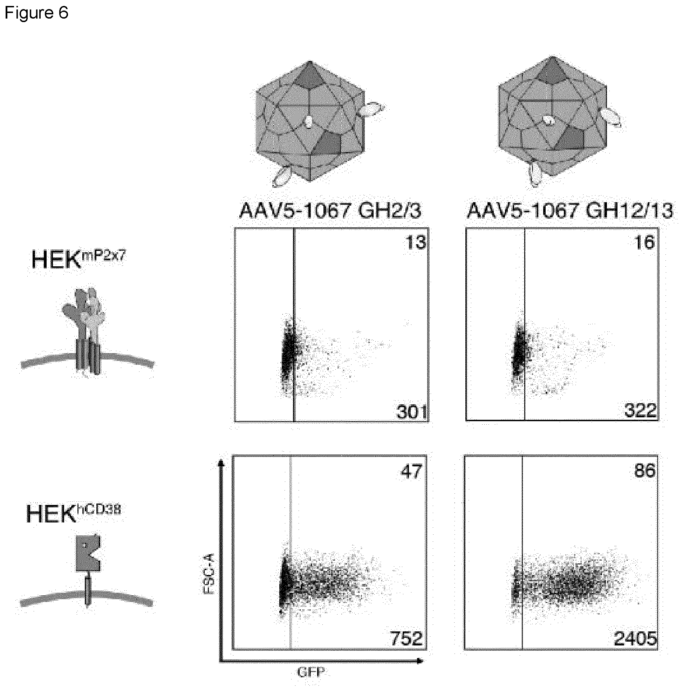

**Figure 6** demonstrates that AAV5 with a CD38-specific nanobody inserted in the GH2/3 or GH12/13 loop transduce CD38-transfected HEK cells (HEK^{CD38}) more efficiently than unrelated P2X7-transfected HEK cells (HEK^{P2X7}).
   HEK cells stably transfected with human CD38 (HEK^{CD38}) or murine P2X7 (HEK^{mP2X7}) were seeded onto a 96-well plate (2×10⁴ cells/well). The cells were incubated with GFP-encoding AAV5 that carry a hCD38-specific nanobody (1067) inserted into the GH2/3 or GH12/13 loop. 48h later the transduction efficiency was analyzed via flow cytometric measurement of GFP expression.

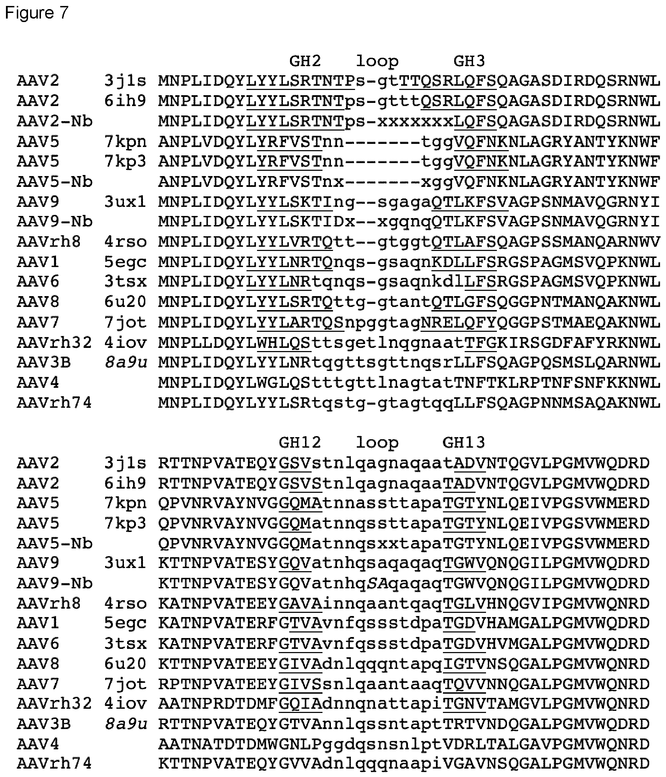

**Figure 7** shows a structure-based alignment of the amino acid sequences of several AAV serotypes at the GH2-GH3 and GH12-GH13 loops flanked by the sequences of the β-strands and some additional amino acid residues. Residues comprising β-strands in the 3D structures (deposited under the indicated pdb code) are underlined, residues comprising the loops are indicated in small letters, amino acids deleted during insertion of the nanobody are indicated by the letter "x". In case of AAV9 GH12-GH13 loop, no amino acids were deleted or exchanged during nanobody insertion. The two residues flanking the insertion site are indicated in italic capital letters (SA).

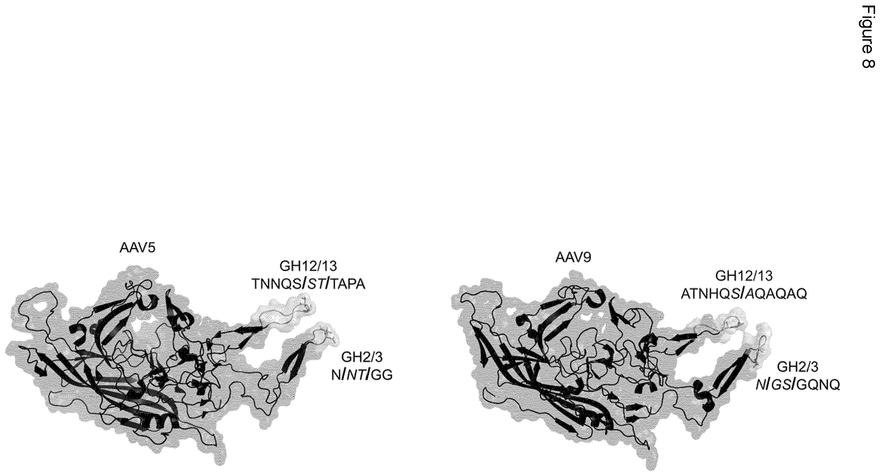

**Figure 8** shows structure models (generated by PyMol) illustrating the two loops extending from the anti-parallel β-strands GH2 and GH3 and GH12 and GH13.

### DETAILED DESCRIPTION OF THE INVENTION

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

For the purposes of the present invention, the term "obtained" is considered to be a preferred embodiment of the term "obtainable". If hereinafter e.g. an antibody is defined to be obtainable from a specific source, this is also to be understood to disclose an antibody which is obtained from this source.

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of ±10%, and preferably of ±5%.

Technical terms are used by their common sense. If a specific meaning is conveyed to certain terms, definitions of terms will be given in the following in the context of which the terms are used.

A first aspect relates to an adeno-associated virus (AAV) capsid fusion protein, wherein the AAV capsid fusion protein comprises a SMBD, in particular a VHH, which is inserted in the GH12-GH13 loop of the AAV capsid protein.

In other words, the invention relates to an adeno-associated virus (AAV) capsid fusion protein, wherein the AAV capsid fusion protein comprises a AAV capsid protein and an insert comprising a binding domain for a cell surface molecule, in particular a SMBD (e.g. VHH), which is inserted in the GH12-GH13 loop of the AAV capsid protein.

The SMBD, may be a VHH, immunoglobulin single variable domain (ISVD), a single chain variable fragment (scFv), a single chain Fab fragment, a variable new antigen receptor (VNAR) domain, or a synthetic or natural binding domain, such as a darpin or the extracellular domain of a membrane protein, for a cell surface molecule. The inventors found that surprisingly these rather large domains can be included into the AAV capsid protein at the G12-G13 loop wherein the capsid is able to assemble fully intact AAVs with enhanced cell targeting properties. Thus, typically the insert as used herein may have a length of at least 50 aa, preferably of 50 aa to 800 aa, 50 aa to 700 aa, 50 aa to 600 aa, 50 aa to 500 aa, 50 aa to 200 aa, 50 aa to 150 aa, 80 aa to 140 aa, 90 aa to 130aa, 100 aa to 120 aa.

In particular, the invention relates to an AAV capsid fusion protein, wherein the AAV capsid fusion protein comprises a SMBD, such as a VHH, which is inserted in the GH12-GH13 loop of the AAV capsid protein. The inventors could show that by using such constructs the transduction of target-expressing cells, i.e. cells expressing a cell surface molecule to which the SMBD entity, such as the VHH entity, of the AAV capsid fusion protein binds, could be increased.

By using the GH12-GH13 loop as insertion site, combining different targeting modifications of the AAV becomes feasible, e.g. the combination of two different SMBDs, the combination of a VHH with another type of a SMBD or the combination of two (or more) VHHs.

In other words, the invention relates to an AAV capsid fusion protein, wherein the AAV capsid fusion protein comprises an AAV capsid protein and a SMBD, such as a VHH, which is inserted in the GH12-GH13 loop of the AAV capsid protein.

Hence, the invention also refers to the combination of at least two SMBDs (e.g. VHHs), such as two to four SMBDs (e.g. VHHs). One specific embodiment refers to a AAV capsid protein which comprises two VHHs. Typically the at least two SMBs (e.g. VHHs) are inserted at different insert sites of the AAV, such as different loops, for example one embodiment refers to a AAV capsid fusion protein wherein one SMBD (e.g.VHH) is inserted in the GH12-G13 loop and a further SMBD (e.g. VHH) is inserted at the GH2-GH3 loop of the AAV. The SMBD (e.g. VHH) inserted in the GH12-GH13 loop and the SMBD (e.g. VHH) inserted in the GH2-GH3 loop may be the same or different. In some embodiments, the SMBD (e.g. VHH) inserted in the GH12-GH13 loop and the SMBD (e.g. VHH) inserted in the GH2-GH3 loop bind to different cell surface molecules.

Another embodiment refer to an AAV capsid fusion protein wherein one SMBD (e.g. VHH) is inserted in the GH12-GH13 loop and a different type of binding domain for a surface molecule is inserted in the GH2-GH3 loop, e.g. an immunoglobulin single variable domain (ISVD), a single chain variable fragment (scFv), a single chain Fab fragment, or a synthetic or natural binding domain for a cell surface molecule.

The invention also refers to embodiments comprising AAV capsid fusion protein wherein one SMBD (e.g. VHH) is inserted in the GH2-GH3 loop and a different type of binding domain for a surface molecule is inserted in the GH12-GH13 loop, e.g. an immunoglobulin (ISVD), a single chain variable fragment (scFv), a single chain Fab fragment, or a synthetic or natural binding domain for a cell surface molecule.

The term "fusion protein" denotes proteins in which at least two polypeptides that originally coded for separate proteins or protein domains are combined in a single molecule. The at least two polypeptides are typically connected by translation of the coding sequence in a cell or cell-free extract into a single polypeptide. In the present case the term refers to a fusion protein in which in the AAV capsid fusion protein sequence comprises a SMBD (e.g. VHH). In other words the SMBD (e.g. VHH) is integrated into the AAV sequence at specific positions which allow the optimal presentation of the SMBD (e.g. VHH) without impairing the structure of the capsid protein and in particular without impairing the structure and function of the AAV in the assembled state.

The term "AAV capsid protein" relates to the three AAV proteins VP-1, VP-2 and VP-3, which together form the capsid of the AAV in the assembled state. In a preferred embodiment the capsid protein is VP-1. In a preferred embodiment the capsid protein is VP-2. Exemplary sequences of AAV capsid proteins are set out in SEQ ID NO: 19 to 57. The skilled person understands that also fragments and variants of said proteins are encompassed, such as sequences which are 70%, 80%, 90% or 95% identical to the sequences of SEQ ID Nos: 19 to 57, in particular fragments and variants which maintain the necessary structure and function for AAV assembly and cell targeting. Thus, the term AAV capsid protein encompasses functional variants and fragments of the AAV capsid protein.

The determination of percent identity between multiple sequences is preferably accomplished using the AlignX application of the Vector NTI AdvanceTM 10 program (Invitrogen Corporation, Carlsbad CA, USA). This program uses a modified Clustal W algorithm (Thompson et al., 1994. Nucl Acids Res. 22: pp. 4673-4680; Invitrogen Corporation; Vector NTI AdvanceTM 10 DNA and protein sequence analysis software. User's Manual, 2004, pp.389-662). The determination of percent identity is performed with the standard parameters of the AlignX application.

Wherever herein reference is made to a variant or fragment sequence having a certain identity to another sequence, it is understood that said variant or fragment remains the function of the original sequence.

Llamas carry a variant immunoglobulin locus that permits the generation of unusual antibodies composed only of heavy chains. The single variable domain of these antibodies (designated VHH, sdAb, or nanobody) is the smallest antigen-binding domain generated by adaptive immune systems. With a long complementarity-determining region 3 (CDR3), VHHs can extend into crevices on proteins that are not accessible to conventional antibodies, including functionally interesting sites such as the active site of an enzyme or the receptor-binding canyon on a virus surface. VHHs offer numerous other advantages compared to conventional antibodies carrying variable domains (VH and VL) of conventional antibodies, including higher stability, solubility, expression yields, and refolding capacity, as well as better in vivo tissue penetration. Since VHH do not bind to VL domains, it is much easier to reformat VHHs into bispecific antibody constructs than constructs containing conventional VH-VL pairs or single domains based on VH domains. The term VHH encompasses functional variants and fragments of the VHHs.

The exemplary VHHs described here were isolated from immunized llama or alpacas. These VHHs derive from VHH frameworks that are frequently observed in both, natural nanobodies and in nanobodies from synthetic libraries. In principle, any nanobody-sequence or variant or fragment thereof, immunoglobulin single variable domain or variant or fragment thereof can be used, provided it exhibits good solubility and specificity for the target molecule.

As set our above, alternatively, scFv or Fab fragments of conventional antibodies could be inserted within the indicated amino acids flanked by β-strands GH2-GH3 and GH12-GH13. Alternatively, other ligand-binding domains, e.g. darpins, VNARs, or the (extracellular domain) ECD of membrane proteins could be inserted within the indicated amino acids flanked by β-strands GH2-GH3 and GH12-GH13.

AAV are small non-enveloped viruses which belong to the family of *Parvoviridae.* The AAV genome consists of single stranded DNA of about 4.7 kilo bases. It comprises T-shaped inverted terminal repeats (ITRs) of 145 bases on each end and carries two open reading frames (ORF): rep and cap, which encode for functional and capsid proteins. The ORF rep encodes for four Rep proteins (Rep78, Rep68, Rep52 and Rep40). Rep 78 and Rep 68 have site-specific, single-strand endonuclease, DNA helicase, and ATP activities, respectively, which are responsible for DNA replication. Rep52 and Rep40 are responsible for packaging of DNA flanked by ITRs into capsids. The ORF cap encodes for three capsid proteins: VP1, VP2 and VP3. Additionally, the VP1 mRNA encodes for the assembly-activating protein (AAP) in a different reading frame. Based in the differences in the amino acid sequences of the capsid proteins, different AAV serotypes are distinguished, i.e. AAV1, AAV2, AAV3, AAV4, AAV5, AAV6, AAV7, AAV8, AAV9, AAV10, AAV11, AAV12, AAVrh10.

Thus, in a specific embodiment the AAV serotype is selected from the group consisting of AAV1, AAV2, AAV3, AAV4, AAV5, AAV6, AAV7, AAV8, AAV9, AAV10, AAV11, AAV12, AAVrh10 or variants thereof or a combination thereof. In one embodiment the AAV serotype is selected from the group consisting of AAV5 and AAV9.

Exemplary variants of the VP proteins may be modified to avoid binding to physiological binding molecules, for example in AAV9 the mutation W503A may be introduced. Another example are mutations reducing binding to heparin sulfate proteoglycan, such as AAV2RA, wherein AAV2 is modified in R585 and R588 mediating binding of AAV2 to heparin sulfate proteoglycan HSPG (Boucas et al. 2009; Münch et al. 2013). The skilled person is also aware of other variants, which are for example modified by the insertion of peptides in the region containing R585 and R588. Other variations are chimeric AAVs, in which the capsid proteins stem from different (e.g. VP1 stemming from AAV and VP2 und VP3 stemming from AAV2), or AAVs in which all capsid proteins contain sequence sections stemming from different AAV serotypes.

Typically, the SMBD (e.g. VHH) is specific for a cell surface molecule, in particular for a cell surface molecule which is specific and/or cell specific, also termed cell surface markers. This means that the marker is not ubiquitously expressed and thereby allows to target specific tissue or cell types while it does not bind to other tissue and cell types. The terms "cell surface molecule" and "cell surface marker" refer to proteins or carbohydrates that are present on the surface of a cell. In a specific embodiment, the cell surface marker is a membrane protein. "Binding to a cell surface marker" as used herein, also means that the SMBD (VHH) is specific for a protein, such as an antibody or an antibody fragment that binds to a specific cell surface marker.

The term "membrane protein" refers to proteins that are attached to or integrated into the membrane of a cell. The term includes integral membrane proteins being permanently anchored to the membrane and peripheral membrane proteins that are temporarily attached to the lipid bilayer or to integral membrane proteins thereof.

The integral membrane proteins may have one or several transmembrane domains. The peripheral membrane proteins may be integrated to the membrane via one or several α-helixes, one or several hydrophobic loops, may be covalently bound to a membrane lipid or interact by electrostatic or ionic interactions with membrane lipids or transmembrane proteins. The membrane protein may be selected from the group consisting of multi-span homo trimer membrane protein, type-II single span membrane protein, GPI-anchor membrane protein, integrins, type I membrane proteins, such as members of the immunoglobulin superfamily, e.g. CD4, receptors, such as cytokine and growth factor receptors, Fc receptors, Toll-like receptors, C-type lektin-like receptors or G-protein coupled receptors.

The term "carbohydrate" particularly refers to glycolipids.

The SMBD (e.g. VHH) may be directed against any membrane protein or membrane glycolipid capable of binding a VHH. In one embodiment the SMBD (e.g. VHH) may be directed against a protein, such as an antibody or an antibody fragment, that specifically binds to a cell surface marker.

The type-II single span membrane protein may be for example CD38. CD38 (cluster of differentiation 38, SEQ ID NO: 33) is prominently expressed by plasma cells and NK cells. CD38 is overexpressed on the surface of many tumor cells, such as multiple myeloma cells. Hence, also encompassed are the fusion proteins comprising a CD38-specific VHH for use as a medicament. More specifically, contemplated are fusion proteins comprising CD38 specific VHHs for the treatment of cancer, such as multiple myeloma.

CD73 (SEQ ID NO: 63), is a cell membrane protein that belongs to the 5'-nucleotidase family. CD73 is a glycosylphosphatidylinositol (GPI) anchored purine salvage enzyme expressed on the surface of human T and B lymphocytes. CD73 is expressed in a wide range of cancer types, including breast cancer, colorectal cancer, glioblastoma, melanoma, prostate cancer, ovarian cancer, and non-small-cell lung cancer (NSCLC).

Programmed death-ligand 1 (PD-L1; SEQ ID NO: 62) is the primary ligand of Programmed cell death -1 (PD-1) and is constitutively expressed on antigen presenting cells, mesenchymal stem cells and bone marrow-derived mast cells. As well as on wide range of tumor cells, including lung cancer, breast cancer and melanoma.

Typically, the SMBD (e.g. VHH) is exposed on the surface of the AAV capsid when the capsid fusion protein is incorporated in the AAV. By the integration in the insertion sites as described herein, e.g. the GH12-GH13 loop or the GH2-GH3 loop exposure of the SMBD (e.g. VHH) on the surface of the AAV capsid may be achieved.

### Linker

One or several linkers may be used in order to facilitate the fixing of the SMBD (e.g. VHH) to the capsid surface and to allow flexible movement of the antigen-binding tip of the VHH away from the capsid surface, i.e. toward the cognate molecule on the surface of the target cell.

Thus, in some embodiments the AAV capsid fusion protein comprises at least one linker linking the SMBD (e.g. VHH) sequence with the VP protein sequence. Typically the length of such a linker (in case of several linkers the length of each linker) is 1 to 100, 2 to 50, 3 to 40, 4 to 30, such as 5 amino acids. Typically, the SMBD (e.g. VHH) is flanked by an N-terminal linker and by a C-terminal linker. The N-terminal and the C-terminal linker may be the same or different. In some embodiments, AAV capsid fusion protein comprises an N-terminal linker, but not a C-terminal linker. The N-terminal linker, may have a length of 1 to 50, preferably 10 to 35, more preferably 15 to 30, most preferably 20 to 25 aa. In particular, the N-terminal linker may facilitate flexible movement of the antigen-binding tip of the VHH away from the capsid surface, i.e. toward the cognate molecule on the surface of the target cell.

The C-terminal linker, may have a length of 1 to 20, preferably 1 to 10, more preferably 2 to 7 amino acids. This linker may fix the base of the SMBD (e.g. VHH) near the capsid surface.

The skilled person is aware of alternative linkers that are suitable for combining two functional polypeptides (Els-Konrath et al. 2001).

In a specific embodiment the N-terminal linker comprises the sequence as set out in SEQ ID NO: 58 or a sequence at least 60%, 70%, 80 %, 90% 95%, 97%, or 99% identical thereto.

In one embodiment the C-terminal linker comprises a sequence selected from the group consisting of SEQ ID NO: 59 or a sequence at least 60%, 70%, 80 %, 90% 95%, 97%, or 99% identical thereto, SEQ ID NO: 64 or a sequence at least 80% identical thereto and SEQ ID NO: 65 or a sequence at least 60%, 70%, 80 %, 90% 95%, 97%, or 99% identical thereto. In one embodiment the C-terminal linker comprises the sequence set out in SEQ ID NO: 59 or a sequence at least 60%, 70%, 80 %, 90% 95%, 97%, or 99% identical thereto.

Thus in a specific embodiment the N-terminal linker comprises the sequence as set out in SEQ ID NO: 58 or a sequence at least 60%, 70%, 80 %, 90% 95%, 97%, or 99% identical thereto and the C-terminal linker comprises the sequence set out in SEQ ID NO: 59 or a sequence at least 60%, 70%, 80 %, 90% 95%, 97%, or 99% identical thereto.

Accordingly, one embodiment refers to an AAV capsid fusion protein wherein one VHH is inserted in the GH12-GH13 loop and a further SMBD (e.g. VHH) is inserted at the GH2-GH3 loop of the capsid protein, wherein both SMBDs (e.g. VHHs) are flanked by an N-terminal and a C-terminal linker.

### Insert

The term "inserted" in particular "wherein the SMBD (e.g. VHH) is inserted in the region" as used herein means, that the SMBD (e.g. VHH) sequence (including optional linkers) can be inserted after any amino acid in said region. By the insertion of the SMBD (e.g. VHH), amino acids from said region may be deleted or modified. Modified, means, that they are substituted without being part of the SMBD (e.g. VHH) sequence. Such modifications may be used for exampled for introduction of restriction sites. In one embodiment, 1, 2, 3, 4, 5, 6, 7, 8, 9, 10, 11 or 12 amino acids may be modified or may be deleted from the GH12-GH13 loop. In particular, 1, 2, 3, 4, 5, 6, 7, 8, 9, 10, 11 or 12 amino acids may be deleted from the GH12-GH13 loop. In one embodiment two amino acids are deleted at the insertion site in the GH12-GH13 loop. In one embodiment, the sequence is introduced between two consecutive amino acids without deletion of amino acids. In one embodiment 1, 2, 3, 4, 5, 6 or 7 amino acids may be modified or may be deleted from the GH2-GH3 loop. In particular 1, 2, 3, 4, 5, 6 or 7 amino acids may be deleted from the GH2-GH3 loop. In one embodiment, the two amino acids flanking the insertion site are deleted upon insertion of the VHH.

The "GH12/13 loop" also "GH12-GH13 loop" or "GH12/13 loop" denotes a loop in the capsid surface which connects the β-strands GH12 and GH13 of the AAV VP protein. For example, the GH12-GH13 loop of the VHH of AAV5 comprises aa 569 to 580, the G12-G13 loop of the VHH of AAV9 comprises aa 581 to 592. Accordingly the, the "GH2/GH3 loop", also "GH2-GH3 loop" or "GH2/3 loop", denotes a loop in the capsid surface which connects the β-strands GH2 and GH3 of the AAV VP protein. For example, the G2-G3 loop of the capsid protein of AAV5 comprises aa 440 to 446, the G2-G3 loop of the capsid protein of AAV9 comprises aa 452 to 458.

### GH12-GH13 insert

Accordingly, one embodiment refers to an AAV capsid fusion protein, wherein the AAV serotype is AAV5 and wherein the SMBD (e.g. VHH) is inserted at any position in the region of aa 569 to 580 of AAV5 VP1 as set out in SEQ ID NO: 31, wherein 1, 2, 3, 4, 5, 6, 7, 8, 9, 10, 11 or 12 amino acids of this region are modified and or deleted. In one embodiment, the SMBD (e.g. VHH) is inserted at any position in the region of aa 575 to 578 of AAV5 VP1 as set out in SEQ ID NO: 31. In one embodiment, the insert is between aa 575 and aa 576 of AAV5 VP1 as set out in SEQ ID NO: 31. In one embodiment, the insert is between aa 575 and aa 576 of AAV5 VP1 as set out in SEQ ID NO: 31, wherein aa 575 and aa 576 are deleted.

One embodiment refers to an AAV capsid fusion protein, wherein the AAV serotype is AAV9 and wherein the SMBD (e.g. VHH) is inserted at any position in the region of aa 581 to 592 of AAV9 VP1 as set out in SEQ ID NO: 43, whereby 0 to 12 amino acids of this region are modified and or deleted. In one embodiment, the SMBD (e.g. VHH) is inserted at any position in the region of aa 586 to 589 of AAV9 VP1 as set out in SEQ ID NO: 43. In a specific embodiment, the insert is between aa 587 to 588 of AAV9 VP1 as set out in SEQ ID NO: 43.

An exemplary embodiment for a constructs in which the VHH is inserted in the GH12-GH13 loop is a AAV capsid fusion protein, wherein the AAV serotype is AAV5, wherein the SMBD (e.g. VHH) is specific for CD38 and the fusion protein has the sequence of SEQ ID NO: 17 or a sequence at least 60%, 70%, 80%, 90%, 95%, 97%, or 99% identical thereto. Figure 6 and shows enhanced transduction of this construct denoted as AAV5_VP1 _GH12/13_1067.

Another embodiment refers to a AAV capsid fusion protein wherein the AAV serotype is AAV9, wherein the VHH is specific for CD73 and wherein the fusion protein has the sequence of SEQ ID NO: 11 or a sequence at least 60%, 70%, 80 %, 90% 95%, 97%, or 99% identical thereto. Figure 6 shows enhanced transduction of construct AAV9_VP1_GH12/13_SB1116.

A further embodiment relates to an AAV capsid fusion protein which contains a CD38 specific VHH and a PD-L1 specific VHH. In one embodiment the CD38 specific VHH is in the GH2-GH3 loop and the PD-L1 specific VHH is in the GH12-GH13 loop.

An exemplary embodiment refers to an AAV capsid fusion protein, wherein the AAV serotype is AAV9, and the capsid fusion protein comprises a VHH specific for CD38 and further a VHH specific for PD-L1. Said AAV capsid fusion protein has the sequence of SEQ ID NO: 13 or a sequence at least 60%, 70%, 80 %, 90% 95%, 97%, or 99% identical thereto. This construct is termed AAV9_VP1GH2/3_370_GH12/13_B3. Figure 4 shows that this construct enhances the transduction of cells expressing CD38, of cells expressing PD-L1 as well as of cells expressing PD-L1 and CD38.

### GH2-GH3 insert

In one embodiment, the SMBD (e.g. VHH) is inserted in GH2-GH3 loop of AAV5 VP1 or the GH2-GH3 loop of AAV9. Providing the specific serotypes of the AAV capsid fusion protein bypasses the need to generate mosaic AAV capsids, where the fusion protein is derived of different AAV serotype as VP2 and VP3. In other words AAV containing all three VP proteins, VP-1, VP-2 and VP-3 from the same serotype, in particular form serotype AAV5 or serotype AAV9, can be provided.

In one embodiment, the SMBD (e.g. VHH) is inserted in the GH2-GH3 loop of AAV5 VP1. In a further embodiment, the SMBD (e.g. VHH) is inserted in the GH2-GH3 loop at any position in the region of aa 440 to 446 of AAV5 VP1 as set out in SEQ ID NO: 31, wherein 0, 1, 2, 3, 4, 5, 6 or 7 amino acids of this region are modified and/or deleted. Specific embodiments relate to an AAV capsid fusion protein, wherein the SMBD (e.g. VHH) is inserted at any position in the region of aa 442 to 444 of AAV5 VP1 as set out in SEQ ID NO: 31. Another embodiment relates to an AAV capsid fusion protein, wherein the SMBD (e.g. VHH) is inserted between aa 442 and aa 443 of AAV5 VP1 as set out in SEQ ID NO: 31.

An exemplary embodiment refers to an AAV capsid fusion protein wherein the SMBD (e.g. VHH) is specific for CD38 and the capsid fusion protein comprises the sequence of SEQ ID NO: 15 or a sequence at least 60%, 70%, 80 %, 90% 95%, 97%, or 99% identical thereto. This construct is denoted AAV5_VP1_GH2/3_1067. Figure 6 shows that this construct enhances transduction.

Another embodiment refers to an AAV capsid fusion protein comprising a SMBD (e.g. VHH) which is inserted in the GH2-GH3 loop of the AAV capsid protein, wherein the AAV serotype is AAV9.

In one embodiment, the SMBD (e.g. VHH) is inserted at any position in the region of aa 452 to 458 of AAV9 VP1 sequence as set out in SEQ ID NO: 43, wherein 0, 1, 2, 3, 4, 5, 6 or 7 amino acids of this region are modified and/or deleted.

In one embodiment, the SMBD (e.g. VHH) is inserted between aa 453 and 456 of AAV9 VP1 sequence as set out in SEQ ID NO: 43.

An exemplary embodiment refers to an AAV capsid fusion protein, wherein the VHH is specific for CD73 and the AAV capsid fusion protein comprises the sequence of SEQ ID NO: 1 or a sequence at least 60%, 70%, 80 %, 90% 95%, 97%, or 99% identical thereto. This construct is denoted AAV9_VP1_GH2/3_SB116. Figure 2 shows that specific transduction of cells expressing CD73 is achieved with this construct.

One embodiment refers to an AAV capsid fusion protein, wherein the VHH is specific for CD38 is introduced in the GH2-GH3 loop. According exemplary embodiments refer AAV capsid fusion protein, wherein the VHH is specific for hCD38 and the AAV capsid fusion protein comprises as sequences which is selected from the group consisting of
a) the sequence of SEQ ID NO: 3 or a sequence at least 60%, 70%, 80%, 90% 95%, 97%, or 99% identical thereto,
b) the sequence of SEQ ID NO: 5 or a sequence at least 60%, 70%, 80%, 90% 95%, 97%, or 99% identical thereto, and
c) the sequence of SEQ ID NO: 7 or a sequence at least 60%, 70%, 80%, 90% 95%, 97%, or 99% identical thereto.

Figure 2 shows that theses constructs enhance the transduction of CD38 expressing cells.

The considerations described here can be used to insert any SMBD (e.g. VHH) or similar ligand into the GH2-GH3 and/or GH12-GH13 loops of any other AAV-capsid, including other serotypes and variants, including but not limited to mutated and reshuffled AAV capsids. The structure-based alignment shown in Figure 7 can be used as a guide to select an insertion site within the indicated amino acid sequences between the two flanking β-strands, i.e. GH2-GH3 and GH12-GH13. I.e. it is likely that the position of the VHH/ligand insertion can be moved by one or more residues without compromising AAV assembly or transduction efficiency.

A further embodiment refers to an AAV capsid fusion protein, wherein the VHH specific for PD-L1 is introduced in the GH2-GH3 loop. An exemplary embodiment refers to a AAV capsid fusion protein, wherein the VHH is specific for PD-L1 and the AAV capsid fusion protein comprises the sequence of SEQ ID NO: 9 or a sequence at least 80% identical thereto. Figure 4 shows that this construct denoted AAV9_VP1_GH2/3_B3 enhances transduction.

The term "target cell" as used herein, refers to a specific cell type or to a group of specific cell types. The target cell may be of endodermal, ectodermal or mesodermal origin. The cell of endodermal origin may be exocrine secretory epithelial cells or a hormone secreting cell. The cell of ectodermal origin may be a cell of the integumentary system or the nervous system. The cell of the mesodermal origin may be a metabolism or storage cell, such as adipocytes, a barrier function cell, such as a lung cell, gut, exocrine glands and urogenital tract cells; extracellular matrix cells, contractile cells, blood and immune system cells, germ cells, nurse cells or interstitial cells. The target cells may also be for example monocytes, T cells or natural killer cells. The target cell may be a tumor cell.

Typically, the VHH is integrated into AAV capsid protein VP2 or VP1 in order to incorporate the VHH into the capsid. During assembly, VP1, VP2, and VP3 are incorporated approximately at a ratio of 1:1:10 into AAV capsids. Preferably the VHH is integrated into VP1 . VP1 - in contrast to VP2 - is required for efficient infection due to the N-terminal phospholipase A2 domain (Girod et al. 2002; Warrington et al. 2004). The fact that VP1 is encoded by a separate messenger RNA, while VP2 and VP3 are encoded by a common mRNA, facilitates the assembly of VHH-containing chimeric capsids of different AAV serotypes.

Specific embodiments relate to a virus containing a AAV capsid fusion protein as described herein. In some embodiments the capsid protein into which the SMBD (e.g. VHH) is integrated is of the same serotype as the capsid proteins into which the SMBD (e.g. VHH) protein is not integrated. In some embodiments the capsid protein into which the SMBD (e.g. VHH) is integrated is of a different serotype than the capsid proteins into which the SMBD (e.g. VHH) protein is not integrated. The inventors showed that surprisingly the capsid protein containing a SMBD (e.g. VHH) could be used in combination with capsid proteins of a different serotype without compromising the transfection efficiency. Surprisingly such mosaic viruses showed a strong transfection efficiency. The usage of a SMBD (e.g. VHH) containing capsid protein of a different serotype allows to easily adapt the whole repertoire of AAV virus serotypes without the need of modifying the capsid proteins for each serotype. Thereby an efficient system for the fast generation of different AAV serotypes specific for a cell surface protein is provided.

For example the capsid protein into which the SMBD (e.g. VHH) is integrated is of the serotype AAV2, while the remaining capsid proteins are of a different serotype, for example a serotype selected from the group consisting of AAV1, AAV3, AAV4, AAV5, AAV6, AAV7, AAV8, AAV9, AAV10, AAV11, AAV12 and AAVrh10. In one embodiment, the capsid protein into which the SMBD (e.g. VHH) is integrated is of the serotype AAV2, or a variation thereof such as AAV2RA, and the other capsid proteins are of a different serotype, e.g. AAV8 or AAV9. In one embodiment, a fusion protein of the VP1 capsid protein into which the VHH domain is integrated is of the serotype AAV2 and the capsid proteins VP2 and VP3 (not containing a VHH domain) are of another serotype (such as serotype AAV8 or AAV9). The skilled person understands that the modular system combining capsid proteins containing VHH domains of one serotype with capsid proteins (not containing a VHH domain) can be transferred to all serotypes.

The sequences of the capsid proteins of the various serotypes are set out in the following:

| AAV serotype | VP1 | VP2 | VP3 |
|---|---|---|---|
| AAV1 | SEQ ID NO: 19 | SEQ ID NO: 20 | SEQ ID NO: 21 |
| AAV2 | SEQ ID NO: 22 | SEQ ID NO: 23 | SEQ ID NO: 24 |
| AAV3 | SEQ ID NO: 25 | SEQ ID NO: 26 | SEQ ID NO: 27 |
| AAV4 | SEQ ID NO: 28 | SEQ ID NO: 29 | SEQ ID NO: 30 |
| AAV5 | SEQ ID NO: 31 | SEQ ID NO: 32 | SEQ ID NO: 33 |
| AAV6 | SEQ ID NO: 34 | SEQ ID NO: 35 | SEQ ID NO: 36 |
| AAV7 | SEQ ID NO: 37 | SEQ ID NO: 38 | SEQ ID NO: 39 |
| AAV8 | SEQ ID NO: 40 | SEQ ID NO: 41 | SEQ ID NO: 42 |
| AAV9 | SEQ ID NO: 43 | SEQ ID NO: 44 | SEQ ID NO: 45 |
| AAV10 | SEQ ID NO: 46 | SEQ ID NO: 47 | SEQ ID NO: 48 |
| AAV11 | SEQ ID NO: 49 | SEQ ID NO: 50 | SEQ ID NO: 51 |
| AAV12 | SEQ ID NO: 52 | SEQ ID NO: 53 | SEQ ID NO: 54 |
| AAVrh10 | SEQ ID NO: 55 | SEQ ID NO: 56 | SEQ ID NO: 57 |

The skilled person understands that also fragments or variants of said proteins are encompassed, in particular sequences which are 70%, 80%, 90% or 95% identical to the sequences of SEQ ID Nos: 19 to 57, in particular fragments and variants which maintain the necessary structure and function for AAV assembly and cell targeting.

In some embodiments, the amino acid sequence of the fusion protein may comprise one or more phenotypically silent substitutions.

"Phenotypically silent substitutions" are also named "conservative amino acid substitutions". The concept of "conservative amino acid substitutions" is understood by the skilled artisan, and preferably means that codons encoding positively-charged residues (H, K, and R) are substituted with codons encoding positively-charged residues, codons encoding negatively- charged residues (D and E) are substituted with codons encoding negatively-charged residues, codons encoding neutral polar residues (C, G, N, Q, S, T, and Y) are substituted with codons encoding neutral polar residues, and codons encoding neutral non-polar residues (A, F, I, L, M, P, V, and W) are substituted with codons encoding neutral non-polar residues. These variations can spontaneously occur, be introduced by random mutagenesis, or can be introduced by directed mutagenesis.

Those changes can be made without destroying the essential characteristics of these polypeptides. The ordinarily skilled artisan can readily and routinely screen variant amino acids and/or the nucleic acids encoding them to determine if these variations substantially reduce or destroy the ligand binding capacity by methods known in the art.

### Nucleotide sequences, vectors, AAV

Another aspect of the invention refers to a nucleotide sequence encoding the fusion protein as described herein.

In the following the nucleotide sequences to the corresponding amino acids are set out for the constructs as disclosed herein.

| **Construct** | **Specificity** | **aa SEQ ID NO:** | **nt SEQ ID NO:** |
|---|---|---|---|
| AAV9_VP1_GH2/3_SB116 | CD73 | 1 | 2 |
| AAV9_VP1_GH2/3_370 | CD38 | 3 | 4 |
| AAV9_VP1_GH2/3_1053 | CD38 | 5 | 6 |
| AAV9_VP1_GH2/3_1067 | CD38 | 7 | 8 |
| AAV9_VP1_GH2/3_B3 | PD-L1 | 9 | 10 |
| AAV9_VP1_GH12/13_SB116 | CD 73 | 11 | 12 |
| AAV9_VP1_GH2/3_370_GH12/13_B3 | CD38 and PD.L1 | 13 | 14 |
| AAV5_VP1_GH2/3_1067 | CD38 | 15 | 16 |
| AAV5_VP1_GH12/13_1067 | CD38 | 17 | 18 |

Moreover, the invention refers to a vector comprising nucleotide sequence as described above.

The skilled person understands, that also the nucleic acid encoding the fusion protein may be modified. Useful modifications in the overall nucleic acid sequence include codon optimization of the sequence. Alterations may be made which lead to conservative substitutions within the expressed amino acid sequence. These variations can be made in complementarity determining and non-complementarity determining regions of the amino acid sequence of the fusion protein that do not affect function. Usually, additions and deletions should not be performed in the CDR3 region. In one embodiment the sequence the AAV capsid fusion protein was codon optimized for expression in HEK cells.

In one embodiment, the AAV capsid fusion protein expression is regulated by a CMV promotor. In a specific embodiment, the vector is condon optimized for expression (in particular for expression in HEK cells) and the AAV capsid fusion protein expression is regulated by a CMV promotor.

In a specific embodiment, the vector comprises the nucleotide sequence coding for the fusion protein comprising a VP1 capsid protein, in which the cell surface marker specific VHH is integrated. The vector may further contain one or several mutations that inhibit the production of mRNA coding for VP2 and VP3.

For the production of an AAV an additional vector could be used, which is mutated, so that VP1 is not expressed, e.g. by a mutation of the VP1 start codon and which therefore codes for VP2 and VP3 but not for VP1.

The skilled person understands that this principle could be also applied for the production of AAVs in which the cell surface marker specific VHH is integrated into VP2 and VP3. Accordingly, in general, a first vector comprises the nucleotide sequence coding for a fusion protein comprising the one type of capsid protein in which the cell surface marker specific VHH is integrated, while the expression of the two other types of capsid proteins is inhibited. A second vector comprises the nucleotide sequence coding for the two other types of capsid proteins, while the expression of the capsid protein, which is modified is inhibited.

Accordingly, the invention also encompasses a kit comprising the vectors as described above.

A "vector" is any molecule or composition that has the ability to carry a nucleic acid sequence into a suitable host cell where synthesis of the encoded polypeptide can take place. Typically, and preferably, a vector is a nucleic acid that has been engineered, using recombinant DNA techniques that are known in the art, to incorporate a desired nucleic acid sequence (e.g. a nucleic acid of the invention). The vector may comprise DNA or RNA and/or comprise liposomes. The vector may be a plasmid, shuttle vector, phagemide, cosmid, expression vector, retroviral vector, lentiviral vector, adenoviral vector, AAV vector or particle and/or vector to be used in gene therapy. A vector may include nucleic acid sequences that permit it to replicate in a host cell, such as an origin of replication. A vector may also include one or more selectable marker genes and other genetic elements known to those of ordinary skill in the art. A vector preferably is an expression vector that includes a nucleic acid according to the present invention operably linked to sequences allowing for the expression of said nucleic acid.

Another aspect of the invention encompasses an AAV containing at least one of the fusion proteins comprising cell surface marker specific VHH and an adeno-associated virus (AAV) capsid protein as described herein.

### Uses

Another aspect of the invention relates to use of the fusion proteins, the nucleotide sequences, the vectors, the AAVs as described herein for improving the transduction efficiency of the AAV targeted cells.

Another aspect of the invention relates to use of the fusion proteins, the nucleotide sequences, the vectors, the AAVs as described herein for introducing a defined nucleic sequence into a target cell.

A further aspect of the invention relates to the fusion proteins, the nucleotide sequences, the vectors, the AAVs as described herein for use as a medicament.

Thus, one embodiment refers to the fusion proteins, the nucleotide sequences, the vectors, the AAVs as described herein for use in gene therapy.

Thus, some embodiments refer to the treatment of genetic deficiencies or genetic disorders. In one embodiment the genetic disorder is selected from the group consisting of retinitis pigmentosa and other forms of blindness, hemophilia and other blood clotting diseases, muscular dystrophy, lysosomal storage diseases. Here nanobody-displaying AAV can aid delivery of functional replacements and /or functional versions of the damaged gene.

In particular, one embodiment refers to the fusion proteins, the nucleotide sequences, the vectors, the AAVs as described herein for use in the treatment of cancer. In one embodiment the cancer is hematopoietic or solid cancer. In exemplary embodiments, the cancer is multiple myeloma, pancreatic carcinoma, lung cancer, prostate cancer, breast cancer, colorectal cancer, glioblastoma, melanoma, ovarian cancer, or non-small-cell lung cancer (NSCLC). In some embodiments the cancer is multiple myeloma, pancreatic carcinoma, lung cancer, prostate cancer.

In one embodiment, the cancer contains cells expressing a protein selected from the group consisting of P2X7, CD38, CD73 and PD-L1. In a specific embodiment the cancer cells express a protein selected from the group consisting of CD38 and CD73.

In particular, one embodiment refers to the fusion proteins, the nucleotide sequences, the vectors, the AAVs as described herein for use in the treatment of neurodegenerative diseases, in particular neurogenerative diseases where cell, such as neurons, astrocytes, microglia in the affected brain region express CD38, CD73 and/or P2X7 or other target antigens recognized by the cognate SMBD (e.g. VHH). Here, SMBD-displaying AAV can enhance delivery of mediators that promote the survival of neurons, prevent formation and/or the deposit of amyloid.

In one embodiment the neurodegenerative disease is selected from the group consisting of Alzheimer, Huntington, Parkinson, Amyotrophic lateral sclerosis or prion's disease.

Some embodiments refer to the fusion proteins, the nucleotide sequences, the vectors, the AAVs as described herein for use in the treatment of chronic infectious diseases, such as those caused by HIV, hepatitis virus, or human papilloma virus. Thereby SMBD (e.g. VHH) displaying AAV can enhance the delivery of virus neutralizing antibodies and /or immune cell mediators.

### Methods

### Generation of VP1-Nb fusion proteins of AAV9

The DNA plasmids used for the production of nanobody-carrying AAV capsids, where a membrane protein-specific nanobody is inserted into prominent surface loops of AAV9, are illustrated schematically in Fig. 1. The VHH is either inserted in the GH2/3 loop between amino acids (aa) 453 and 455, in the GH12/13 loop between aa 587 and 588 or simultaneously in both positions of the viral protein 1 (VP1). To allow an easy exchange of the inserted nanobody, the VHH-coding sequence is flanked by restriction sites for the endonucleases *Pcil* and *Notl.* The genes encoding the respective VP1-VHH fusion proteins were designed *in silico* using a codon-optimization tool for expression in human cells. At both insertion sites, the VHH is flanked by a restriction site for the endonuclease *NgoMIV* and a 25 aa long flexible peptide linker at the N-Terminus and a 5 aa long peptide linker and a restriction site for the endonuclease *Kasl* at the C-Terminus to allow an outward orientation of the antigen-binding paratope of the VHH. The optimized nucleotide sequences were ordered as synthetic gene fragments. Prior to cloning the *pRC_RR* vector was digested with *Accl* and religated, thereby removing the REP sequence and one of two *Xbal* restriction sites. The VP1-VHH-encoding gene blocks were cloned via restriction sites for the endonucleases *Swal* and *Xbal* into the *pRC_RR* vector downstream of the p40 promotor and via restriction sites for the endonucleases *BamHI* and *Xbal* into the pcDNA3.1 vector downstream of a CMV promotor. The gene blocks and 2 µg of the target vectors were digested with 1 µl of the respective restriction enzymes (NEB Biolabs) for 5 h at 37 °C according to the manufacturers protocol. 2 µl of the ligation samples were transformed into competent *E*. *coli* bacteria. Plasmid DNA was extracted using the QIAprep Spin Miniprep Kit (QIAGEN). DNA sequences were verified by sequencing.

### Production of AAV9 containing VP1-Nb fusion proteins

The production of AAVs was performed by triple or quadruple transfection of HEK293AAV cells (Cell Biolabs) with three (AAV9) or four (AAV9-Nb^{VP1(GH2/3)}, AAV9-Nb^{VP1(GH12/13)}) plasmids. For transfection, 7 × 10⁶ cells/dish were plated in 25 ml DMEM complete medium with 10 % FCS in 15 cm cell culture dishes (Cellstar, Greiner Bio-One) and cultured at 37 °C, 5 % CO₂ until 70 % confluency. The medium was replaced by 17 ml of fresh medium 1 h before transfection. Polyethyleneimine (25kD, PEI, Polysciences Inc.) was used as transfection reagent at a concentration of 0.333 mg/ml diluted in ddH₂O. The following plasmids were used for the production of AAV9: 10 µg *pRC_AAV9* encoding rep of AAV2 and *cap* of AAV9 (provided by Dirk Grimm, University of Heidelberg), 15 µg helper plasmid *pHelper* (Plasmid Factory) and 7.5 µg of the transgene *scAAV-CMVeGFP* (provided by Martin Trepel, UKE Hamburg) per culture dish. For the production of AAV9 containing VP1-Nb fusion proteins, 12 µg *pcDNA3.1_VP1-Nb* or *pRC_RR_AAV9_VP1-Nb* and 7.5 µg *pRC_AAV9_VP2-3* were used instead of *pRC-AAV9.* The plasmid *pRC_AAV9_VP2-3* was generated by mutation of the sixth codon of VP1 to a stop codon (provided by Dirk Grimm, University of Heidelberg). The corresponding plasmids were mixed in 750 µl ddH₂O before the addition of 750 µl 300 mM NaCl and mixing by vortex for 10 s. PEI was added for each transfection in fourfold molar excess to the DNA. Therefore, PEI was diluted in 750 µl ddH₂O and was also supplemented with 750 µl 300 mM NaCl and mixed for 10 s by vortex. The PEI solution was added dropwise to the DNA solution, vortexed for 10 s and incubated at room temperature for 15-20 min. The mix (3 ml) was added dropwise uniformly to the cells of one culture dish. The cells were cultured at 37 °C and 5 % CO₂. After 24 h, the medium was replaced with 25 ml of fresh medium. After three days, AAVs were harvested from the cell supernatant and cell lysates.

### Recovery of recombinant AAVs from cell culture supernatants and cell lysates

Transfected HEK293AAV cells were carefully detached from the culture dish with a cell scraper three days after medium exchange and were transferred with the culture supernatant into a 50 ml falcon tube and centrifuged for 5 minutes at 1200 rpm, 4° C. The supernatant was transferred to a fresh 50 ml falcon tube and the cell pellet was stored on ice until the cells were lysed. For the precipitation of AAVs from the supernatant, 10 g PEG-8000 and 5.8 g NaCl were added per 100 ml supernatant (about 10 % w/v PEG-8000 and 1 M NaCl) and incubated rolling overnight at 4 °C. The next day, the suspensions were centrifuged at 4,600 rpm for 30 min. The supernatant was discarded, and the pellet-containing tubes were again centrifuged for 3 min at 4,600 rpm in order to completely remove remaining supernatant. The AAV-containing pellet was resuspended in 1 ml PBS-MK (PBS with 1 mM MgCl₂ and 2.5 mM KCl₂). For the recovery of AAVs from cell lysates, cell pellets were resuspended in 1 ml PBS-MK and transferred to 15 ml falcon tubes. Cells were lysed by three cycles of freeze/thaw (5-10 min freezing in dry ice surrounded by 99% ethanol, 5-10 minutes thawing in a 37 °C water bath). Subsequently, DNA and RNA in supernatants and lysates were digested using the endonuclease benzonase (Sigma, E1014, 250 U/µl) for 30 min at 37 °C (50 U/ml supernatant or lysate). Remaining insoluble material was eliminated by centrifugation for 15 min at 4,600 rpm. Viral genome titers were determined by quantitative real-time PCR. Lysates and supernatants were stored at 80 °C.

### Transduction of HEK cells with AAV9-Nb^{VP1(GH2/3)} and AAV9-Nb^{VP1(GH12/13)}

To evaluate the transduction of AAV9 containing VP1 fusion proteins with inserted CD73- or CD38-specific VHH SB116 and 1067, respectively, HEK293 cells stably transfected with CD73 or CD38 were seeded in 96-well cell culture plates (2×10⁴ cells/well) in DMEM complete medium with 10 % FCS. PEG-precipitated cell culture supernatants (10 µl/well) containing GFP-encoding AAVs were added to the cells. 48 h later the expression of the transgene GFP was analyzed by flow cytometry using the FACS Canto II and FlowJo software (BD Biosciences) (Fig. 2). AAVs that carry nanobodies in the GH2/3 loop (AAV9-1067^{VP1(GH2/3)}, AAV9-SB116^{VP1(GH2/3)}) or GH12/13 loop (AAV9-SB116^{VP1(GH12/13)}) enhance the transduction of HEK cells expressing the cognate membrane protein 20-100fold compared to the respective unrelated HEK cells. Use of the *pcDNA3.1* plasmids during the production of AAVs carrying VP1-Nb fusion proteins, results in an even stronger enhancement in transduction of the target cells compared to AAV carrying VP1-Nb fusion proteins produced with the *pRC_RR* plasmids.

### Detection of VP1-Nb fusion proteins incorporated into AAV9

For detecting Nb-VP1 fusion proteins incorporated into AAV9 capsids, proteins in 15 µl PEG-precipitated supernatants from AAV productions were size-fractionated by SDS-PAGE and transferred to a PVDF membrane. The detection of capsid proteins was carried out with the mAb B1 (Wistuba et al. 1995) (provided as hybridoma supernatant by Jakob Körbelin, UKE), that recognizes an epitope at the C-terminus of all three capsid proteins (Fig. 3). Both VP1-Nb fusion proteins, where the nanobody is either inserted in the GH2/3 or the GH12/13 loop, show the expected shift in MW of ~15 kDa compared to the parental VP1 (100 kDa vs 85 kDa).

### Transduction of HEK cells with AAV9-Nb^{VP1(GH2/3+GH12/13)}

To evaluate the transduction of AAV9 containing VP1 fusion proteins with inserted CD38- and PD-L1-specific VHH 370 and B3, HEK cells stably transfected with CD38 and untransfected HEK cells were transiently transfected with murine PD-L1 and were seeded in 96-well cell culture plates (2×10⁴ cells/well) in DMEM complete medium with 10% FCS. PEG-precipitated cell culture supernatants containing GFP-encoding AAVs were added to the cells at 1,000 vg/cell (AAV9-370), 2,000 vg/cell (AAV9-B3) or 10,000 vg/cell (AA9-370_B3). 48 h later the expression of the transgene GFP was analysed by flow cytometry using the FACS Canto II and FlowJo software (BD Biosciences) (Fig. 4). Cells expressing CD38 alone or CD38 and PD-L1 were more efficiently transduced with AAV9-370 and AAV9-370_B3 than P2X7-expressing control cells. Vice versa, cells expressing PD-L1 alone or CD38 and PD-L1 were transduced more efficiently with AAV9-B3 or AAV9-370_B3 than the control cells, demonstrating the bi-specificity of AAV9 containing VP1 fusion proteins with a nanobody inserted in both loops (AAV9_370_B3).

### Generation of VP1-Nb fusion proteins of AAV5

The DNA plasmids used for the production of nanobody-carrying AAV capsids, where a membrane protein-specific nanobody is inserted into prominent surface loops of AAV5, are illustrated schematically in Fig. 5. The VHH is either inserted in the GH2/3 loop replacing two amino acids (aa) at position 443 and 444 or in the GH12/13 loop replacing two aa at position 576 and 577 of the viral protein 1 (VP1). To allow an easy exchange of the inserted nanobody, the VHH-coding sequence and a flexible peptide linker of 25 aa at the N-Terminus and 5 aa at the C-Terminus is flanked by restriction sites for the endonucleases *NgoMIV* and *Kasl.* This allows an outward orientation of the antigen-binding paratope of the VHH. The genes encoding the respective VP1-VHH fusion proteins were designed *in silico* using a codon-optimization tool for expression in human cells. The optimized nucleotide sequences were ordered as synthetic gene fragments. The VP1-VHH-encoding gene blocks were cloned via restriction sites for the endonucleases *Swal* and *Notl* into the *pRC_RR* vector downstream of the p40 promotor. The gene blocks and 2 µg of the target vector were digested with 1 µl of the respective restriction enzymes (NEB Biolabs) for 1 h at 37 °C according to the manufacturers protocol. 2 µl of the ligation samples were transformed into competent *E. coli* bacteria. Plasmid DNA was extracted using the QIAprep Spin Miniprep Kit (QIAGEN). DNA sequences were verified by sequencing.

### Production of AAV5 containing VP1-Nb fusion proteins

The production of AAV5 containing VP1-Nb fusion proteins (AAV5-Nb^{VP1(GH2/3)}, AAV5-Nb^{VP1(GH12/13)}) was performed by quadruple transfection of HEK293AAV cells (Cell Biolabs) with four plasmids. For transfection, 7 × 10⁶ cells/dish were plated in 25 ml DMEM complete medium with 10 % FCS in 15 cm cell culture dishes (Cellstar, Greiner Bio-One) and cultured at 37 °C, 5 % CO₂ until 70 % confluency. The medium was replaced by 17 ml of fresh medium 1 h before transfection. Polyethyleneimine (25kD, PEI, Polysciences Inc.) was used as transfection reagent at a concentration of 0.333 mg/ml diluted in ddH₂O. The following plasmids were used for the production of AAV5 containing VP1-Nb: 4.5 µg *pRC_AAV5_VP-Nb* and 4.5 µg *pRC_AAV5_VP2-3* encoding rep of AAV2 and *VP2-VP3* of AAV5 (Addgene, #104964), 15 µg helper plasmid *pHelper* (Plasmid Factory) and 7.5 µg of the transgene *scAAV-CMVeGFP* (provided by Martin Trepel, UKE Hamburg) per culture dish. The plasmid *pRC_AAV5_VP2-3* was generated by mutation of the VP1-start codon ATG to an AAG codon. The corresponding plasm ids were mixed in 750 µl ddH₂O before the addition of 750 µl 300 mM NaCl and mixing by vortex for 10 s. PEI was added for each transfection in fourfold molar excess to the DNA. Therefore, PEI was diluted in 750 µl ddH₂O and was also supplemented with 750 µl 300 mM NaCl and mixed for 10 s by vortex. The PEI solution was added dropwise to the DNA solution, vortexed for 10 s and incubated at room temperature for 15-20 min. The mix (3 ml) was added dropwise uniformly to the cells of one culture dish. The cells were cultured at 37 °C and 5 % CO₂. After 24 h, the medium was replaced with 25 ml of fresh medium. After three days, AAVs were harvested from the cell supernatant and cell lysates. Recombinant AAV5 were recovered from cell culture supernatants and cell lysates following the same protocol as for AAV9.

### Transduction of HEK cells with AAV5-1067^{VP1(GH2/3)} and AAV5-1067^{VP1(GH12/13)}

HEK cells stably transfected with CD38 or P2X7 were seeded in 96-well cell culture plates (2×10⁴ cells/well) in DMEM complete medium with 10% FCS. Cells lysates containing GFP-encoding AAVs were added to the cells at 200 vg/cell. 48 h later the expression of the transgene GFP was analysed by flow cytometry using the FACS Canto II and FlowJo software (BD Biosciences) (Fig. 6). The results show that the transduction of HEK cells expressing the target CD38 (HEK^{hCD38}) is 3 to 5-fold more efficiently compared to control HEK cells (HEK^{mP2X7}).

The description further contains the following embodiments:
1. Adeno-associated virus (AAV) capsid fusion protein, wherein the AAV capsid fusion protein comprises a AAV capsid protein and a surface marker binding domain (SMBD) which is inserted in the GH12-GH13 loop of the AAV capsid protein.
2. Adeno-associated virus (AAV) capsid fusion protein according to claim 1, wherein the SMBD is selected from the group consisting of a VHH, immunoglobulin single variable domain (ISVD), single chain variable fragment (scFv), single chain Fab fragment, a synthetic or natural binding domain for a cell surface molecule.
3. Adeno-associated virus (AAV) capsid fusion protein according to claim 1, wherein the SMBD is a VHH.
4. AAV capsid fusion protein comprising at least one VHH which is inserted in the AAV capsid protein, preferably, comprising two to four VHHs which are inserted in the AAV capsid protein, more preferably comprising two VHHs which are inserted in the AAV capsid protein.
5. AAV capsid fusion protein according to any one of the preceding embodiments, wherein the SMBD is specific for a cell surface molecule.
6. AAV capsid fusion protein according to any one of the preceding embodiments, wherein the SMBD is exposed on the surface of the AAV capsid when the capsid fusion protein is incorporated in the AAV.
7. AAV capsid fusion protein according to any one of the preceding embodiments, wherein the AAV capsid fusion protein further comprises at least one linker.
8. AAV capsid fusion protein according to any one of embodiments 7, wherein each linker has a length of 1 to 100, 2 to 50, 3 to 40, 4 to 30, such as 5 amino acids.
9. AAV capsid fusion protein according to any one of embodiments 7 or 8, wherein the SMBD is flanked by an N-terminal and a C-terminal linker.
10. AAV capsid fusion protein according to embodiment 9, wherein the N-terminal linker has a length of 1 to 45, preferably 10 to 35, more preferably 15 to 30, most preferably 20 to 25 aa.
11. AAV capsid fusion protein according to any one of embodiments 9 or 10, wherein the N-terminal linker comprises the sequence as set out in SEQ ID NO: 58 or a sequence at least 80 % identical thereto.
12. AAV capsid fusion protein according to any one of embodiments 9 to 111, wherein the C-terminal linker is has a length of 1 to 20, preferably 1 to 10, more preferably 2 to 7 amino acids.
13. AAV capsid fusion protein according to any one of embodiments 9 to 12, wherein the C-terminal linker comprises a sequence selected from the group consisting of (i) SEQ ID NO: 59 or a sequence at least 80% identical thereto, (ii)SEQ ID NO: 64 or a sequence at least 80% identical thereto and (iii) SEQ ID NO: 65 or a sequence at least 80% identical thereto.
14. AAV capsid fusion protein according to any one of embodiments 9 to 13, wherein the C-terminal linker comprises the sequence set out in SEQ ID NO: 59 or a sequence at least 80% identical thereto.
15. AAV capsid fusion protein according to any one of the preceding embodiments, wherein the SMBD is inserted in the region corresponding to aa 584 to 591 of AAV9 as set out in SEQ ID NO: 43.
16. AAV capsid fusion protein according to any one of the preceding embodiments, wherein the SMBD is inserted in the region corresponding to aa 587 and 588 of AAV9 as set out in SEQ ID NO: 43.
17. AAV capsid fusion protein according to any one of the preceding embodiments, wherein the AAV capsid protein sequence comprises a SMBD which is inserted in the GH2-GH3 loop of the AAV capsid protein.
18. AAV capsid fusion protein according to any one of the preceding embodiments, wherein the SMBD is selected from the group of a VHH, immunoglobulin single variable domain, scFv, Fab, a synthetic or natural binding domain for a cell surface molecule.
19. AAV capsid fusion protein according to any one of embodiments 17 or 19, wherein the SMBD is a VHH.
20. AAV capsid fusion protein according to any one of the preceding embodiments, wherein the capsid protein is selected from the group consisting of VP1, VP2 or VP3.
21. AAV capsid fusion protein according to any one of the preceding embodiments, wherein the capsid protein is VP1.
22. AAV capsid fusion protein according to any one of the preceding embodiments, wherein the AAV capsid protein is selected from the group consisting of AAV1 capsid protein, AAV2 capsid protein, AAV3 capsid protein, AAV4 capsid protein, AAV5 capsid protein, AAV6 capsid protein, AAV7 capsid protein, AAV8 capsid protein, AAV9 capsid protein, AAV10 capsid protein, AAV11 capsid protein, AAV12 capsid protein, AAVrh10 capsid protein or variants thereof or a combination thereof.
23. AAV capsid fusion protein according to embodiment 22, wherein AAV capsid protein is AAV5 capsid protein or AAV9 capsid protein.
24. AAV capsid fusion protein according to embodiment 23, wherein the AAV serotype is AAV5 and wherein the VHH is inserted at any position in the region of aa 569 to 580 of AAV5 VP1 as set out in SEQ ID NO: 31, wherein 0-12 amino acids of this region are modified and/or deleted.
25. AAV capsid fusion protein according to embodiment 24, wherein the AAV serotype is AAV5 and wherein the VHH is inserted at any position in the region of aa 573 to 580 of AAV5 VP1 as set out in SEQ ID NO: 31.
26. AAV capsid fusion protein according to embodiment 25, wherein VHH is inserted in the region of aa 575 to 578 of AAV5 VP1 as set out in SEQ ID NO: 31.
27. AAV capsid fusion protein according to embodiment 26, wherein the insert is between aa 575 and aa 576 of AAV5 VP1 as set out in SEQ ID NO: 31.
28. AAV capsid fusion protein according to any one of the preceding embodiments, wherein the VHH is specific for CD38.
29. AAV capsid fusion protein according to any one of embodiments 23 to 28,
   wherein the AAV serotype is AAV5, wherein the VHH is specific for CD38 and the AAV capsid fusion protein has the sequence of SEQ ID NO: 17 or a sequence at least 80% identical thereto.
30. AAV capsid fusion protein according to embodiment 23, wherein the AAV serotype is AAV 9.
31. AAV capsid fusion protein according to embodiment 30, wherein the AAV serotype is AAV9 and wherein the VHH is inserted at any position in the region of aa 581 to 592 of AAV9 VP1 as set out in SEQ ID NO: 43, wherein 0-12 amino acids of this region are modified and/or deleted.
32. AAV capsid fusion protein according to embodiment 31, wherein the VHH is inserted at any position in the region of aa 584 to 591 of AAV9 VP1 as set out in SEQ ID NO: 43.
33. AAV capsid fusion protein according to embodiment 32, wherein the VHH is inserted in the region of aa 586 to 589 of AAV9 VP1 as set out in SEQ ID NO: 43.
34. AAV capsid fusion protein according to embodiment 33, wherein the insert is between aa 587 to 588 of AAV9 VP1 as set out in SEQ ID NO: 43.
35. AAV capsid fusion protein according to any one of the preceding embodiments, wherein the VHH is specific for CD73.
36. AAV capsid fusion protein according to embodiment 35, wherein the AAV serotype is AAV9, wherein the VHH is specific for CD73 and wherein the AAV capsid fusion protein has the sequence of SEQ ID NO: 11 or a sequence at least 80% identical thereto.
37. AAV capsid fusion protein according to any one of the preceding embodiments, wherein the AAV capsid fusion protein comprises a VHH specific for PD-L1 and a VHH specific for CD38.
38. AAV capsid fusion protein according to embodiment 37, wherein the AAV serotype is AAV9, wherein the AAV capsid fusion protein comprises a VHH specific for PD-L1 and a VHH specific for CD38 and has the sequence of SEQ ID NO: 13 or a sequence at least 80% identical thereto.
39. AAV capsid fusion protein comprising a VHH which is inserted in the GH2-GH3 loop of the AAV capsid protein, wherein the AAV serotype is AAV5.
40. AAV capsid fusion protein according to embodiment 39, wherein the VHH is inserted at any position in the region of aa 440 to 446 of AAV5 VP1 as set out in SEQ ID NO: 31, wherein 0-7 amino acids of this region are modified and/or deleted.
41. AAV capsid fusion protein according to embodiment 40, wherein the VHH is inserted in the region of aa 442 to 444 of AAV5 VP1 as set out in SEQ ID NO: 31.
42. AAV capsid fusion protein according to embodiment 41, wherein the VHH is inserted between aa 442 and aa 443 of AAV5 VP1 as set out in SEQ ID NO: 31.
43. AAV capsid fusion protein according to any one of embodiments 39 to 42, wherein the VHH is specific for CD38 and the capsid fusion protein comprises the sequence of SEQ ID NO: 15 or a sequence at least 80% identical thereto.
44. AAV capsid fusion protein comprising a VHH which is inserted in the GH2-GH3 loop of the AAV capsid protein, wherein the AAV serotype is AAV9.
45. AAV capsid fusion protein according to embodiment 44, wherein the VHH is inserted at any position in the region in the region of aa 452 to 458 of AAV9 VP1 sequence as set out in SEQ ID NO: 43, wherein 0-7 amino acids of this region are modified and/or deleted.
46. AAV capsid fusion protein according to embodiment 45, wherein the VHH is inserted between aa 453 and 456 of AAV9 VP1 sequence as set out in SEQ ID NO: 43.
47. AAV capsid fusion protein according any one of embodiments 44 to 46, wherein the VHH is specific for CD73 and the AAV capsid fusion protein comprises the sequence of SEQ ID NO: 1 or a sequence at least 80% identical thereto.
48. AAV capsid fusion protein according any one of embodiments 44 to 46,wherein the VHH is specific for hCD38 and the AAV capsid fusion protein comprises as sequences which is selected from the group consisting of
   a) the sequence of SEQ ID NO: 3 or a sequence at least 80% identical thereto,
   b) the sequence of SEQ ID NO: 5 or a sequence at least 80% identical thereto, and
   c) the sequence of SEQ ID NO: 7 or a sequence at least 80% identical thereto.
49. AAV capsid fusion protein according any one of embodiments 44 to 46,wherein the VHH is specific for PD-L1 and the AAV capsid fusion protein comprises the sequence of SEQ ID NO: 9 or a sequence at least 80% identical thereto.
50. Nucleotide sequence encoding the AAV capsid fusion protein according to any one of embodiments 1 to 49.
51. Vector comprising nucleotide sequence according to embodiment 50.
52. AAV comprising the AAV capsid fusion protein according to embodiment 1 to 49.
53. The AAV according to embodiment 52 further comprising a transgene.
54. Use of the AAV capsid fusion protein according to any one of the claims 1 to 49, the nucleotide sequence according to claim 50, the vector according to embodiment 51, the AAV according to claim 52 or 53 for improving the transduction efficiency of the AAV targeted cells.
55. Use AAV capsid fusion protein according to any one of the embodiments 1 to 49, the nucleotide sequence according to embodiment 50, the vector according to embodiment 51, the AAV according to claim 52 or 53 for introducing a defined nucleic sequence into a target cell.

### REFERENCES

Bartlett et al. (1999) Targeted adeno-associated virus vector transduction of nonpermisive cells mediated by a bispecific F(ab'gamma)2 antibody. Nat Biotechnol, 17:181-6.
Boucas et al. (2009) Engineering andeno-associated virus serotype 2-based targeting vectors using a new insertion site position 453- and single point mutations. J Gene Med 11:1103-13
Els-Conrath et al. (2001) Camel singledomain antibodies as modular building units in bispecific and bivalent antibody constructs. J Biol Chem, 276: 7346-50.
Girod et al. (2002) The VP1 capsid protein of adeno-associated virus type 2 is carrying a phospholipase A2 domain required for virus infectivity. J Gen Virol. 83(Pt5):973-8.
Judd et al. (2012) Random Insertion of mCherry Into VP3 Domain of Adeno-associated Virus Yields Fluorescent Capsids With no Loss of Infectivity. Mol Ther Nucleic Acids 1: e54.
Münch et al. (2013) Displaying high-affinity ligands on adeno-associated viral vectors enables tumor cell-specific and safe gene transfer. Mol Ther 21:109-18
Nagy et al. (2003). Manipulating the Mouse Embryo: A Laboratory Manual. Cold Spring Harbor Laboratory Press, New York.
Warrington et al. (2004) Adeno-associated virus type 2 VP2 capsid protein is nonessential and can tolerate large peptide insertions at its N terminus. J Virol. 78(12):6595-609.
Wistuba et al. (1995) Intermediates of adeno-associated virus type 2 assembly: identification of soluble complexes containing Rep and Cap proteins. J Virol. 1995 Sep;69(9):5311-9.

## Claims

1. Adeno-associated virus (AAV) capsid fusion protein, wherein the AAV capsid fusion protein comprises an AAV capsid protein and a surface marker binding domain (SMBD) which is inserted in the GH12-GH13 loop of the AAV capsid protein.

2. Adeno-associated virus (AAV) capsid fusion protein according to claim 1, wherein SMBD is selected from the group of a VHH, immunoglobulin single variable domain (ISVD), single chain variable fragment (scFv), single chain Fab fragment, a synthetic or natural binding domain for a cell surface molecule.

3. Adeno-associated virus (AAV) capsid fusion protein according to claim 1 or 2,
wherein the SMBD is a VHH.

4. AAV capsid fusion protein according to any one of the preceding claims, wherein the AAV capsid protein sequence further comprises a SMBD which is inserted in the GH2-GH3 loop of the AAV capsid protein.

5. AAV capsid fusion protein according to claim 4, wherein the SMBD which is inserted in the GH2-GH3 loop is a VHH.

6. AAV capsid fusion protein according to any one the preceding claims, wherein the AAV capsid protein is selected from the group consisting of AAV1 capsid protein, AAV2 capsid protein, AAV3 capsid protein, AAV4 capsid protein, AAV5 capsid protein, AAV6 capsid protein, AAV7 capsid protein, AAV8 capsid protein, AAV9 capsid protein, AAV10 capsid protein, AAV11 capsid protein, AAV12 capsid protein, AA Vrh1 0 capsid protein or variants thereof or a combination thereof, preferably wherein AAV capsid protein is AAV5 capsid protein or AAV9 capsid protein.

7. AAV capsid fusion protein according to claim 6, wherein the AAV serotype is AAV5 and wherein the VHH is inserted at any position in the region of aa 569 to 580 of AAV5 VP1 as set out in SEQ ID NO: 31, wherein 0 to 12 amino acids of this region are modified and/or deleted.

8. AAV capsid fusion protein according to any one of claims 1 to 6, wherein the AAV serotype is AAV5, wherein the VHH is specific for CD38 and the AAV capsid fusion protein has the sequence of SEQ ID NO: 17 or a sequence at least 80% identical thereto.

9. AAV capsid fusion protein according to claim 8, wherein the AAV serotype is AAV9 and wherein the VHH is inserted at any position in the region of aa 581 to 592 of AAV9 VP1 as set out in SEQ ID NO: 43, wherein 0 to 12 amino acids of this region are modified and/or deleted.

10. AAV capsid fusion protein according to any one of claims 1 to 6, wherein the AAV serotype is AAV9, wherein the VHH is specific for CD73 and wherein the fusion protein has the sequence of SEQ ID NO: 11 or a sequence at least 80% identical thereto.

11. AAV capsid fusion protein according to any one of claims 1 to 6, wherein the AAV serotype is AAV9, wherein the fusion protein comprises a VHH specific for PD-L1 and a VHH specific for CD38 and has the sequence of SEQ ID NO: 13 or a sequence at least 80% identical thereto.

12. Nucleotide sequence encoding the fusion protein according to any one of claim 1 to 11.

13. Vector comprising nucleotide sequence according to claim 12.

14. AAV comprising the capsid fusion protein according to claim 13.

15. Use AAV capsid fusion protein according to any one of the claims 1 to 11, the nucleotide sequence according to claim 12, the vector according to claim 13, the AAV according to claim 14 for introducing a defined nucleic sequence into a target cell.
